# EUROPEAN PATENT APPLICATION

(11) **EP 4 245 325 A1**
(43) Date of publication of application: **20.09.2023**
(21) Application number: 21891860.5
(22) Date of filing: 09.11.2021
(51) Int. Cl.: A61L 9/00, A61L 9/01, A61L 9/015, F24F 7/003

(54) **AIR CLEANING SYSTEM AND AIR CLEANING METHOD**

(30) Priority: 13.11.2020 JP 2020189387
(71) Applicant: IHI Corporation, Tokyo 135-8710 (JP)
(72) Inventor: TAKAHASHI Katsumi, Tokyo 135-8710 (JP); MUROFUSHI Shoko, Tokyo 135-8710 (JP); ISHII Kosuke, Tokyo 135-8710 (JP)
(74) Representative: TBK
(86) International application number: PCT/JP2021/041190
(87) International publication number: WO 2022/102614

(57) **Abstract**

Provided is an air cleaning system (1) including: an ozone generation unit (40) that generates ozone gas; and a discharge unit (50) that atomizes or vaporizes an aqueous alcohol solution and discharges the aqueous alcohol solution. A mole fraction of an alcohol included in the aqueous alcohol solution with respect to the aqueous alcohol solution is 0.05 or more and 0.35 or less. A hydroxyl radical is generated through a reaction of the ozone gas with the aqueous alcohol solution that has been atomized or vaporized.

## Description

### TECHNICAL FIELD

The present disclosure relates to an air cleaning system and an air cleaning method.

### BACKGROUND ART

Air cleaners using filters such as HEPA filters to purify indoor spaces are being sold. However, such air cleaners are unable to sterilize or inactivate microorganisms such as viruses and bacteria. Meanwhile, since ozone gas has a strong oxidizing power, it is used to sterilize or inactivate microorganisms such as viruses and bacteria present in spaces and on the surface of objects. However, ozone gas in high concentrations is known to be harmful to human health, and the Japan Society for Occupation Health has recommended that the permissible ozone concentration be 0.1 ppm or less. Meanwhile, when the concentration of ozone gas in a room is 0.1 ppm or less, the effect of sterilizing or inactivating microorganisms is small, and thus fumigation is performing using ozone gas having a high concentration when a person is not in the room as described in Patent Literature 1.

Patent Literature 1 discloses an air cleaner that includes a first vent communicating with an interior space, an ozone generation unit, an ozone decomposition unit, and a second vent communicating with the interior space. The ozone generation unit is arranged between the first vent and the ozone decomposition unit. A blocking means is provided between the ozone generation unit and the ozone decomposition unit, which can be opened and closed and can block the flow of ozone gas when closed.

### CITATION LIST

### PATENT LITERATURE

Patent Literature 1: Japanese Unexamined Patent Application Publication No. 2002-126063

### SUMMARY OF THE INVENTION

In conventional air cleaners, when a person is in a room, the blocking means is opened to decompose ozone gas in the ozone decomposition unit. In addition, in conventional air cleaners, the blocking means is closed when a person is not in the room, and odors and sources of odors in the interior space are removed using ozone gas generated in the ozone generation unit. However, in conventional air cleaners, ozone gas having a high concentration is not available when a person is in the room. Thus, if an infected person having an infectious disease is in the room, droplets containing infectious agents such as the above-described microorganisms are dispersed into the interior space due to coughing and the like, and the droplets adhere to and accumulate on the surface of objects, thereby increasing the risk of infection from the infected person to others.

An object of the present disclosure is to provide an air cleaning system and an air cleaning method that are capable of purifying air using a hydroxyl radical generated by adding an additive to ozone gas.

### SOLUTION TO PROBLEM

An air cleaning system according to the present disclosure includes an ozone generation unit that generates ozone gas, and a discharge unit that atomizes or vaporizes an aqueous alcohol solution and discharges the aqueous alcohol solution. A mole fraction of an alcohol included in the aqueous alcohol solution with respect to the aqueous alcohol solution is 0.05 or more and 0.35 or less. A hydroxyl radical is generated through a reaction of the ozone gas with the aqueous alcohol solution that has been atomized or vaporized.

The alcohol may include at least one of ethanol or isopropanol. The alcohol may be discharged in such a manner that the alcohol, when converted as gas, is 12 times or less the ozone gas in volume ratio. The air cleaning system may further include a first housing that houses the ozone generation unit and the discharge unit, wherein the ozone gas and the aqueous alcohol solution that has been atomized or vaporized may react inside the first housing. The air cleaning system may further include a first housing that houses the ozone generation unit and the discharge unit, wherein the ozone gas and the aqueous alcohol solution that has been atomized or vaporized may react outside the first housing. The air cleaning system may further include a second housing that houses the ozone generation unit, and a third housing that houses the discharge unit, wherein the ozone gas and the aqueous alcohol solution that has been atomized or vaporized may react outside the second housing and the third housing. An ozone gas concentration in an external space may be controlled to be less than or equal to 0.1 ppm.

An air cleaning method according to the present disclosure includes a step of generating ozone gas, a step of atomizing or vaporizing an aqueous alcohol solution and discharging the aqueous alcohol solution, and a step of generating a hydroxyl radical through a reaction of the ozone gas with the aqueous alcohol solution that has been atomized or vaporized. A mole fraction of an alcohol included in the aqueous alcohol solution with respect to the aqueous alcohol solution is 0.05 or more and 0.35 or less.

The present disclosure can provide an air cleaning system and an air cleaning method that are capable of purifying air using a hydroxyl radical generated by adding an additive to ozone gas.

### BRIEF DESCRIPTION OF DRAWINGS

[FIG. 1] FIG. 1 is a schematic diagram illustrating an air cleaning system according to one embodiment.
[FIG. 2] FIG. 2 is a schematic diagram illustrating an air cleaning system according to another embodiment.
[FIG. 3] FIG. 3 is a schematic diagram illustrating an air cleaning system according to another embodiment.
[FIG. 4] FIG. 4 is a graph illustrating a relationship between an alcohol mole fraction and a hydroxyl radical fluorescence peak height.

### DESCRIPTION OF EMBODIMENTS

Some exemplary embodiments are described below with reference to the drawings. Note that dimensional ratios in the drawings are exaggerated for convenience of the description and may differ from the actual ratios.

### [First Embodiment]

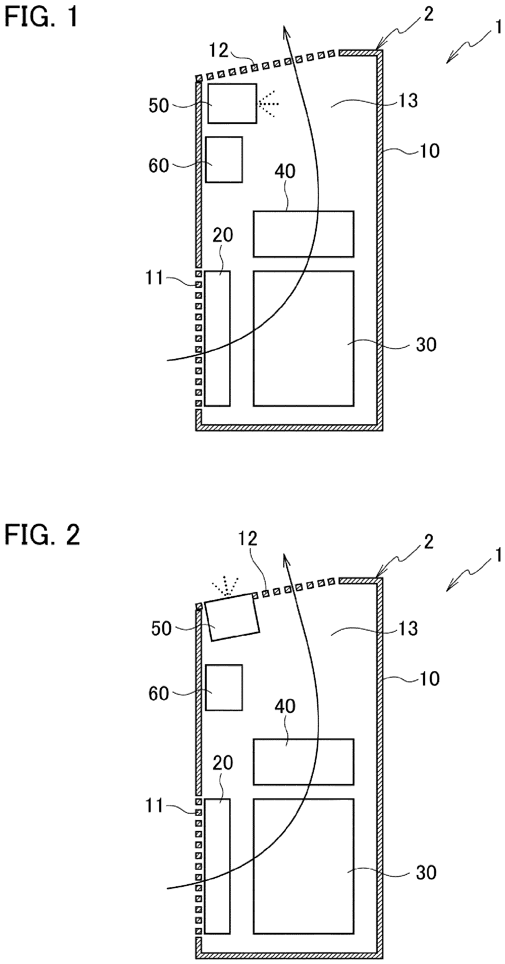

First, an air cleaning system 1 according to a first embodiment will be described using FIG. 1. As illustrated in FIG. 1, the air cleaning system 1 according to the present embodiment includes an air cleaning device 2. The air cleaning device 2 includes a first housing 10, a filter unit 20, a blower unit 30, an ozone generation unit 40, a discharge unit 50, and a control unit 60. However, it is sufficient that the air cleaning device 2 include the ozone generation unit 40 and the discharge unit 50, and the air cleaning device 2 does not necessarily need to include the first housing 10, the filter unit 20, the blower unit 30, and the control unit 60. In the air cleaning system 1 according to the present embodiment, as described below, ozone gas generated in the ozone generation unit 40 reacts with an aqueous alcohol solution as an additive discharged from the discharge unit 50 to generate a hydroxyl radical. Then, the air cleaning system 1 cleans the air using the hydroxyl radical.

Since the redox potential of ozone (O₃) is as high as 2.07 V and ozone gas exhibits an extremely strong oxidizing power, ozone gas is used for sterilization and virus inactivation. In addition, the redox potential of a hydroxyl radical (OH⁻) is even higher at 3.85 V, which is generated in the air through a reaction of part of ozone gas, and a hydroxyl radical has the strongest oxidizing power among molecules including oxygen atoms. However, under normal conditions in which an additive is not added, a hydroxyl radical is unlikely to be generated from ozone gas. Thus, in the air cleaning system 1 according to the present embodiment, the unreacted part of ozone gas is made to react with an aqueous alcohol solution, which is an additive, to generate a hydroxyl radical having a high oxidizing power, and the concentration of the hydroxyl radical in the space is increased to improve the sterilization power.

The first housing 10 houses the filter unit 20, the blower unit 30, the ozone generation unit 40, the discharge unit 50, and the control unit 60. The first housing 10 divides the internal space of the air cleaning device 2 from the interior space, which is the external space. The first housing 10 is provided with an intake port 11 for taking in air from the outside of the first housing 10 and an exhaust port 12 for discharging the air taken in from the intake port 11 to the outside of the first housing 10. An air flow path 13 connecting the intake port 11 and the exhaust port 12 is provided inside the first housing 10, and air flows through the air flow path 13 from the intake port 11 toward the exhaust port 12. In the air flow path 13, the filter unit 20, the blower unit 30, and the ozone generation unit 40 are arranged in this order from the upstream side.

The filter unit 20 is provided at the intake port 11 to remove foreign matter from the air taken in from the outside of the first housing 10. The filter unit 20 may include, for example, a known dustproof filter. The filter unit 20 may also include a known functional filter in addition to the dustproof filter.

The blower unit 30 is arranged downstream of the filter unit 20 in the air flow path 13. The blower unit 30 allows air to flow from the outside of the first housing 10 to the inside of the first housing 10 through the intake port 11, and discharges the air flowing into the first housing 10 to the outside of the first housing 10 through the exhaust port 12. The blower unit 30 may include, for example, a sirocco fan. Although the blower unit 30 is arranged downstream of the filter unit 20 in the drawings, if it is arranged in the air flow path 13, it can generate airflow from the intake port 11 to the exhaust port 12. Thus, the blower unit 30 may be arranged near the exhaust port 12, for example.

The ozone generation unit 40 generates ozone gas. The ozone gas is gaseous ozone. Specifically, the ozone generation unit 40 generates ozone gas from oxygen in the air taken in from the outside of the first housing 10. The ozone gas generated by the ozone generation unit 40 is discharged to the outside of the first housing 10 through the exhaust port 12. The ozone generation unit 40 is not limited as long as it can generate ozone gas, and may include a discharge-type ozone generation device for corona discharge, silent discharge, or the like, for example, or an ultraviolet lamp type ozone generation device. The amount of ozone gas generated in the ozone generation unit 40 can be adjusted by changing the output, the ozone generation time, or the like of the ozone generation unit 40, for example.

The air cleaning system 1 may include an ozone concentration sensor that measures the ozone gas concentration at least on either the outside or inside of the first housing 10. When the ozone gas concentration outside the first housing 10 is measured, the ozone gas concentration in the room after the ozone gas and the aqueous alcohol solution have reacted is measured, so that a direct index of the amount of ozone gas in the room can be obtained. In contrast, when the ozone gas concentration inside the first housing 10 is measured, the ozone concentration sensor is arranged on the downstream side of the ozone generation unit 40 in the air flow path 13, for example. In this case, since the ozone concentration sensor can directly measure the concentration of the ozone gas generated in the ozone generation unit 40, an index of the amount of ozone gas generated in the ozone generation unit 40 can be obtained. Thus, the amount of ozone gas generated can be easily adjusted according to a state of the ozone generation unit 40.

The ozone gas concentration in the external space of the air cleaning system 1 is preferably controlled to be 0.1 ppm or less. Thus, even when a person is in a room, the air can be cleaned by the air cleaning system 1. The ozone gas concentration in the external space may be controlled to be 0.08 ppm or less. Since the air cleaning system 1 can sterilize using a hydroxyl radical, the lower limit of the ozone gas concentration in the external space is not limited, but it may be controlled to be 0.01 ppm or more, for example. Note that in this description, ppm means parts per million volume.

The concentration of ozone gas generated in the ozone generation unit 40 is not limited. As described above, the concentration of ozone gas in the external space is preferably 0.1 ppm or less. However, since ozone gas reacts with an aqueous alcohol solution to generate a hydroxyl radical, at least part of the ozone gas generated in the ozone generation unit 40 disappears by reacting with the aqueous alcohol solution. Thus, the concentration of the ozone gas generated in the ozone generation unit 40 may exceed 0.1 ppm. However, from the viewpoint of further reducing the ozone gas concentration in the atmosphere outside the first housing 10, it is preferable that the concentration of ozone gas generated in the ozone generation unit 40 be controlled to be 0.1 ppm or less. In addition, by setting the ozone gas concentration to 0.1 ppm or less, deterioration of certain materials due to ozone gas can be suppressed. The concentration of ozone gas generated in the ozone generation unit 40 may be controlled to be 0.08 ppm or less. Since the air cleaning system 1 can purify air using a hydroxyl radical, the lower limit of the concentration of ozone gas generated in the ozone generation unit 40 is not limited, but it may be controlled to be 0.01 ppm or more, for example.

The discharge unit 50 atomizes or vaporizes an aqueous alcohol solution and discharges it. Then, the ozone gas generated in the ozone generation unit 40 reacts with the atomized or vaporized aqueous alcohol solution to generate a hydroxyl radical. At this time, the atomized or vaporized aqueous alcohol solution may be supplied to the ozone gas, or the ozone gas may be supplied to the atomized or vaporized alcohol.

A hydroxyl radical is a type of radical that can, for example, deprive hydrogen from organic matter constituting microorganisms such as proteins and lipids, decompose them, and sterilize bacteria or inactivate viruses. A hydroxyl radical has a redox potential higher than that of ozone and has a sufficient bactericidal or inactivating effect even when the concentration of a hydroxyl radical is low. For example, when comparing a CT value (concentration-time value) required to sterilize 99% of Escherichia coli, it is said that a hydroxyl radical has a CT value that is less than several hundredths of that of ozone.

Therefore, in the air cleaning system 1 according to the present embodiment, through the action of a hydroxyl radical, it is possible to effectively sterilize bacteria and inactivate viruses even when the ozone gas concentration is low. In addition, a hydroxyl radical is highly reactive and combines with hydrogen it has taken away and changes into water, and thus has low persistence. Furthermore, a hydroxyl radical can decompose organic matter using its high oxidizing power, and thus it can be applied not only to sterilization of bacteria and inactivation of viruses, but also to deodorization, bleaching, and washing.

Note that it is said that the CT value required to kill 99% of Escherichia coli using a hydroxyl radical is 4.7 × 10⁻¹⁰ mol/L·min, and the same tendency is expected to occur in other bacteria and viruses. Meanwhile, when the ozone gas concentration is 0.1 ppm and the relative humidity is 80% (equivalent to what is called ozone fog), TCID50 of the novel coronavirus (SARS-CoV-2) decreases to 27% at CT (ppm × min) = 24, 13% at CT = 42, and 4.6% at CT = 60. When these value are approximated with an exponential function, it can be inferred that TCID50 is 1% when CT is about 92. That is, the 99% inactivation time at an ozone gas concentration of 0.1 ppm is 920 minutes or about 15 hours. As in the example described below, in the case in which an additive is added to gas having an ozone gas concentration of 0.1 ppm and the amount of a hydroxyl radical is twice as large as the amount when moisture is added, the time required for 99% inactivation is approximately halved, in other words, becomes about 7.5 hours. This inactivation time is inversely proportional to the amount of activation (real hydroxyl radical concentration) of the reaction product of ozone gas and an additive. By controlling the amounts of ozone gas and an additive in this way, it is possible to estimate the time until 99% inactivation of the novel coronavirus. In addition, when ozone fog formed by mixing ozone gas and water vapor (fine particles of water) is used, the same sterilization rate is exhibited in about 1/5 of the time when ozone gas from which moisture has been removed is used. When ozone gas is reacted with an aqueous alcohol solution, sterilization takes about 1/2 of the time when ozone fog is used, which means that the time until inactivation is about 1/10 of the time when ozone gas from which moisture is removed is used.

The discharge unit 50 atomizes or vaporizes an aqueous alcohol solution and discharges it as described above. The aqueous alcohol solution may be discharged through either atomization or vaporization, or through atomization and vaporization. In addition, the aqueous alcohol solution may be minute droplets immediately after atomization, and then minute droplets may be vaporized. The atomization or vaporization of the aqueous alcohol solution facilitates the reaction between ozone gas and an aqueous alcohol solution, which enables a hydroxyl radical to be efficiently generated. The discharge unit 50 may include at least one of a sprayer for atomizing an aqueous alcohol solution or a vaporizer for vaporizing an aqueous alcohol solution. The sprayer may include at least one selected from the group consisting of an ultrasonic sprayer, a mesh sprayer, and a compressor sprayer, for example. The vaporizer may include a heat vaporizer, for example.

The ultrasonic sprayer sprays an aqueous alcohol solution using the cavitation effect of ultrasonic waves. The ultrasonic sprayer may include an outer tank provided with an ultrasonic vibrator, and an inner tank provided inside the outer tank. For example, the ultrasonic sprayer contains in the outer tank a cooling liquid, such as water, in contact with the inner tank, and the ultrasonic vibration energy from the ultrasonic vibrator is transmitted through the cooling liquid to the aqueous alcohol solution contained in the inner tank, which enables the aqueous alcohol solution to be sprayed.

The mesh sprayer uses ultrasonic vibration and a mesh to spray an aqueous alcohol solution. The mesh sprayer may include an ultrasonic vibrator, a mesh having multiple openings, and a tank containing an aqueous alcohol solution. In the mesh sprayer, for example, an aqueous alcohol solution in the tank is pushed out of multiple openings in the mesh through the vibration of the ultrasonic vibrator. Thus, the mesh sprayer can spray the aqueous alcohol solution.

The compressor sprayer sprays an aqueous alcohol solution using gas compressed by the compressor. The compressor sprayer may include a spray nozzle, a tank, and a compressor. The aqueous alcohol solution contained in the tank is sent to a spray nozzle, and the compressor pressure-feeds gas, such as air, to the spray nozzle. The compressor sprayer can spray the aqueous alcohol solution from the spray nozzle using the gas fed by the compressor.

The heat vaporizer heats an aqueous alcohol solution and discharges the aqueous alcohol solution vaporized through heating. The heat vaporizer may include a heater and a tank to store the aqueous alcohol solution. In the heat vaporizer, the aqueous alcohol solution stored in the tank may be fed to the heater, and the fed aqueous alcohol solution may be vaporized through heating. The heater may heat the aqueous alcohol solution using Joule heat.

A mole fraction of an alcohol included in the aqueous alcohol solution with respect to the aqueous alcohol solution is 0.05 or more and 0.35 or less. A hydroxyl radical can also be generated by causing ozone gas to react with water, but using an aqueous alcohol solution having a mole fraction within the above-described range increases the amount of a hydroxyl radical generated by causing ozone gas to react with the aqueous alcohol solution. Thus, a hydroxyl radical having a higher oxidizing power than that of ozone gas can be generated with high efficiency, and thus it is possible to improve the bacteria sterilization effect and the virus inactivation effect even when the ozone gas concentration in the room remains low. The mole fraction of an alcohol in the aqueous alcohol solution may be 0.1 or more and may be 0.3 or less.

An alcohol may be discharged in such a way that the alcohol, when converted as gas, is 12 times or less the ozone gas in volume ratio. When the above-described volume ratio is 12 times or less, the amount of a hydroxyl radical generated through the reaction of ozone gas with an aqueous alcohol solution may increase. The above-described volume ratio may be 11 times or less. The above-described volume ratio may be 0.1 times or more, 1 times or more, or 2 times or more.

The alcohol included in an aqueous alcohol solution is not particularly limited. From the point of view of easily atomizing or vaporizing the aqueous alcohol solution, it is preferable to include an alcohol having a carbon number of 1 to 5. The aqueous alcohol solution may include at least one alcohol selected from the group consisting of methanol, ethanol, propanol such as isopropanol, butanol, and pentanol. Among these, the alcohol preferably contains at least one of ethanol or isopropanol, both of which are commonly used as a rubbing alcohol. In addition, an alcohol preferably contains ethanol from the viewpoint that the amount of a hydroxyl radical generated increases.

In the air cleaning system 1 according to the present embodiment, ozone gas reacts with atomized or vaporized aqueous alcohol solution inside the first housing 10. By causing ozone gas and an aqueous alcohol solution to react inside the first housing 10, part of ozone gas disappears inside the first housing 10. Thus, even if a large amount of ozone gas is generated, it is possible to suppress the discharge of highly concentrated ozone gas into the room. The discharge unit 50 may discharge an aqueous alcohol solution into the air flow path 13 or discharge an aqueous alcohol solution into a mixing chamber different from the air flow path 13, and cause the ozone gas to react with the aqueous alcohol solution in the air flow path 13 or in the mixing chamber, for example. The discharge unit 50 may discharge the aqueous alcohol solution downstream of the ozone generation unit 40 in the air flow path 13 as illustrated in FIG. 1, or may discharge it upstream of the ozone generation unit 40 in the air flow path 13.

The control unit 60 includes a CPU, a ROM, and a RAM. The CPU reads a program stored in the ROM and can execute instructions such as operation and control according to the program. The RAM stores information acquired from the blower unit 30, the ozone generation unit 40, the discharge unit 50, and the like, and the CPU can read the information stored in the RAM and use it for processing such as operation. The control unit 60 may control the on and off of the driving of the blower unit 30, the ozone generation unit 40, and the discharge unit 50. The control unit 60 may perform control in such a manner that the blower unit 30, the ozone generation unit 40, and the discharge unit 50 are continuously driven, or may perform control in such a manner that they are intermittently driven at a predetermined timing. The driving timings of the blower unit 30, the ozone generation unit 40, and the discharge unit 50 may be the same or different from each other. For example, the control unit 60 may control the driving timing of the ozone generation unit 40 and the discharge unit 50 in such a manner that production of ozone gas and discharge of an aqueous alcohol solution occur simultaneously. The control unit 60 may also control the driving timings of the ozone generation unit 40 and the discharge unit 50 in such a manner that production of ozone gas and discharge of an aqueous alcohol solution occur at different timings. The control unit 60 may also control the driving of the ozone generation unit 40 based on a concentration measured using the ozone concentration sensor.

Note that the present embodiment describes an example in which a hydroxyl radical generated through a reaction between ozone gas generated in the ozone generation unit 40 and an aqueous alcohol solution discharged from the discharge unit 50 is discharged from the exhaust port 12 without passing through an ozone decomposition unit or the like. However, since the air is purified using a hydroxyl radical in the air cleaning system 1 according to the present embodiment, the air cleaning system 1 may include an ozone decomposition unit that decomposes the unreacted ozone gas and is arranged downstream of a region where the hydroxyl radical is generated in the air flow path 13. This enables the ozone gas concentration in the room to be reduced while the air is purified. The ozone decomposition unit may include an ozonolysis catalyst, such as manganese dioxide, that decomposes ozone gas through contact with the ozone gas.

As described above, the air cleaning system 1 according to the present embodiment includes the ozone generation unit 40 that generates ozone gas and the discharge unit 50 that atomizes or vaporizes an aqueous alcohol solution and discharges it. The mole fraction of an alcohol included in an aqueous alcohol solution with respect to the aqueous alcohol solution is 0.05 or more and 0.35 or less. A hydroxyl radical is generated through a reaction between ozone gas and an atomized or vaporized aqueous alcohol solution.

Also, an air cleaning method according to the present embodiment includes a step of generating ozone gas, a step of atomizing or vaporizing an aqueous alcohol solution and discharging the atomized or vaporized aqueous alcohol solution, and a step of generating a hydroxyl radical through a reaction between ozone gas and the atomized or vaporized aqueous alcohol solution. The mole fraction of an alcohol included in the aqueous alcohol solution with respect to the aqueous alcohol solution is 0.05 or more and 0.35 or less.

Since a hydroxyl radical has a higher oxidizing power than that of ozone gas, it is possible to effectively sterilize bacteria and inactivate viruses even when the concentration of the hydroxyl radical in the space is low. Thus, it is not necessary to perform fumigation using ozone gas having a high concentration when a person is not in the room, as with the conventional air cleaner. A hydroxyl radical can also be applied to deodorization, bleaching, washing, and the like instead of or in addition to bacteria sterilization and virus inactivation, because it can decompose organic matter using its high oxidizing power. Thus, in the air cleaning system 1 or the air cleaning method according to the present embodiment, it is possible to purify the air using a hydroxyl radical generated by adding an additive to ozone gas. Note that the air cleaning system 1 or the air cleaning method may use ozone gas having a high concentration in combination with a hydroxyl radical to improve bacterial sterilization and virus inactivation when a person is not in the room.

### [Second embodiment]

Next, an air cleaning system 1 according to a second embodiment will be described using FIG. 2. The air cleaning system 1 according to the first embodiment includes the first housing 10 housing the ozone generation unit 40 and the discharge unit 50, and ozone gas reacts with an atomized or vaporized aqueous alcohol solution inside the first housing 10. The air cleaning system 1 according to the present embodiment is common with the air cleaning system 1 according to the first embodiment in that it includes the first housing 10 housing the ozone generation unit 40 and the discharge unit 50. Meanwhile, in the air cleaning system 1 according to present embodiment, ozone gas reacts with an atomized or vaporized aqueous alcohol solution outside the first housing 10. In other respects, the air cleaning system 1 according to the second embodiment is the same as the air cleaning system 1 according to the first embodiment.

As illustrated in FIG. 2, the discharge unit 50 is provided in the first housing 10 in such a manner that an aqueous alcohol solution can be discharged outside the first housing 10. The discharge unit 50 discharges the aqueous alcohol solution toward ozone gas discharged through the exhaust port 12 of the first housing 10, for example. Consequently, the ozone gas generated in the ozone generation unit 40 and the aqueous alcohol solution discharged from the discharge unit 50 are mixed outside the first housing 10, and a hydroxyl radical is generated.

As described above, the air cleaning system 1 according to the present embodiment includes the first housing 10 housing the ozone generation unit 40 and the discharge unit 50, and ozone gas reacts with an atomized or vaporized aqueous alcohol solution outside the first housing 10. According to the air cleaning system 1 according to the present embodiment, since a hydroxyl radical is generated outside the first housing 10, it can contribute to sterilization or inactivation of bacteria and viruses attached to an indoor wall, a floor, and a ceiling as well as an object placed in the room.

### [Third embodiment]

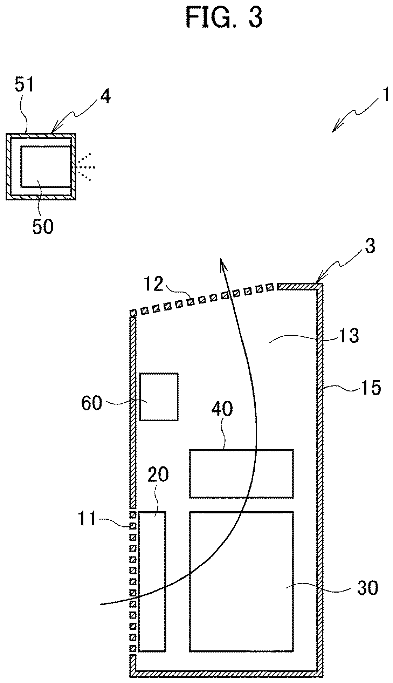

Next, an air cleaning system 1 according to a third embodiment will be described using Fig. 3. The air cleaning system 1 according to the first embodiment includes the first housing 10 housing the ozone generation unit 40 and the discharge unit 50. In contrast, the air cleaning system 1 according to the present embodiment includes a second housing 15 housing the ozone generation unit 40, and a third housing 51 housing the discharge unit 50, instead of the first housing 10. Ozone gas reacts with an atomized or vaporized aqueous alcohol solution outside the second housing 15 and the third housing 51. In other respects, the air cleaning system 1 according to the third embodiment is the same as the air cleaning system 1 according to the first embodiment.

The air cleaning system 1 according to the present embodiment includes an ozone generation device 3 and a discharge device 4. The ozone generation device 3 includes the second housing 15, the filter unit 20, the blower unit 30, the ozone generation unit 40, and the control unit 60. The second housing 15 houses the filter unit 20, the blower unit 30, the ozone generation unit 40, and the control unit 60. The second housing 15 corresponds to the first housing 10 and divides the internal space of the ozone generation device 3 from the interior space, which is the external space. The second housing 15 is provided with the intake port 11 and the exhaust port 12 as in the first housing 10, and the air flow path 13 connecting the intake port 11 and the exhaust port 12 is provided inside the second housing 15. The ozone generation device 3 differs from the air cleaning device 2 according to the first embodiment in that it does not include the discharge unit 50, but is the same as the air cleaning device 2 in other respects. Note that it is sufficient that the ozone generation device 3 include the ozone generation unit 40, and the ozone generation device 3 does not necessarily need to include the filter unit 20, the blower unit 30, and the control unit 60.

The discharge device 4 includes the discharge unit 50 and the third housing 51. The discharge unit 50 is housed in the third housing 51. The discharge unit 50 is provided in the third housing 51 and sprays an aqueous alcohol solution outside the third housing 51. The same discharge unit 50 can be used as in the first embodiment, and atomizes or vaporizes an aqueous alcohol solution and discharges it.

Ozone gas generated in the ozone generation unit 40 is discharged outside the second housing 15. The discharge device 4 discharges an aqueous alcohol solution outside the third housing 51. Then, the ozone gas generated in the ozone generation unit 40 and the aqueous alcohol solution discharged from the discharge unit 50 are mixed outside the second housing 15 and the third housing 51 to generate a hydroxyl radical.

As described above, the air cleaning system 1 according to the present embodiment further includes the second housing 15 housing the ozone generation unit 40, and the third housing 51 housing the discharge unit 50. Ozone gas reacts with an atomized or vaporized aqueous alcohol solution outside the second housing 15 and the third housing 51. In the air cleaning system 1 according to the present embodiment, ozone gas reacts with an aqueous alcohol solution outside the second housing 15 and the third housing 51. Thus, a hydroxyl radical is generated outside the second housing 15 and the third housing 51, which can contribute to sterilization or inactivation of bacteria and viruses attached to an indoor wall, a floor, and a ceiling as well as an object placed in the room. Since the second housing 15 housing the ozone generation unit 40 and the third housing 51 housing the discharge unit 50 can be separated, maintenance in the event of failure can be facilitated.

The air cleaning system 1 is installed, for example, in a room of a building, a moving body, and the like, and can purify indoor air. The air cleaning system 1 can be used in, for example, a house, a hospital, a school, a factory, a smoking room, a car, a bus, a train, a ship, and the like.

Although the air cleaning system 1 has been described in the first to third embodiments as being portable and movable, the air cleaning system 1 may be a fixed air cleaning system installed on a ceiling, a wall, or the like in a room.

### EXAMPLES

The embodiments will be described in more detail below with examples and comparative examples, but the embodiments are not limited to these examples.

### [Example 1]

First, special grade isopropanol manufactured by FUJIFILM Wako Pure Chemical Corporation and water were mixed at a ratio of 1:9 in such a manner that the mole fraction of the isopropanol was 0.1 to prepare an isopropanol aqueous solution as a chemical agent (additive). Then, ozone gas and the isopropanol aqueous solution, which has been vaporized, are reacted in a reaction tank in such a manner that the mixing ratio of ozone and isopropanol was about 1:2.8 (volume ratio) when converted as gas. Specifically, the isopropanol was discharged in such a manner that the isopropanol, when converted as gas, was about 2.8 times the ozone gas in volume ratio. More specifically, the above-described chemical agent discharged at a flow rate of 20 µL/min was vaporized through heating. The vaporized chemical agent and gas having an ozone gas concentration of 500 ppm and a flow rate of 500 mL/min were mixed and reacted in a reaction tank kept at 30 °C.

A beaker containing a chemical sensor liquid was placed in the reaction tank. The chemical sensor liquid was prepared by dissolving 0.084 g of terephthalic acid in 250 mL of a mixed liquid of DMF (N, N-dimethylformamide) : methanol = 4:1 in such a manner that the concentration of the terephthalic acid was 2 mmol/L. The terephthalic acid used was special grade terephthalic acid manufactured by FUJIFILM Wako Pure Chemical Corporation, the DMF used was special grade DMF manufactured by FUJIFILM Wako Pure Chemical Corporation, and the methanol used was special grade methanol manufactured by FUJIFILM Wako Pure Chemical Corporation.

Terephthalic acid does not emit fluorescence when irradiated with ultraviolet light having a wavelength of 310 nm. However, 2-hydroxyterephthalic acid, generated through a reaction between terephthalic acid and a hydroxyl radical, absorbs ultraviolet light having a wavelength of 310 nm and emits fluorescence having a peak around 425 nm. Thus, this chemical sensor liquid was used as an index of the amount of a hydroxyl radical generated. The fluorescence intensity of the chemical sensor liquid was measured using a spectrofluorometer RF-5300 manufactured by SHIMADZU CORPORATION. Specifically, the chemical sensor liquid was irradiated with ultraviolet light having a wavelength of 310 nm, and the peak intensity (hydroxyl radical fluorescence peak height) of the spectrum of the fluorescence emitted from the chemical sensor liquid at around 425 nm was measured.

### [Example 2]

The hydroxyl radical fluorescence peak height was measured in the same manner as in example 1 except that an isopropanol aqueous solution as a chemical agent was prepared by mixing the isopropanol and water at a ratio of 3:7 in such a manner that the mole fraction of the isopropanol was 0.3. Note that the isopropanol was discharged in such a manner that the isopropanol, when converted as gas, was about 8.1 times the ozone gas in volume ratio.

### [Example 3]

The hydroxyl radical fluorescence peak height was measured in the same manner as in example 1 except that an ethanol aqueous solution as a chemical agent was prepared by mixing special grade ethanol manufactured by FUJIFILM Wako Pure Chemical Corporation and water at a ratio of 1:9 in such a manner that the mole fraction of the ethanol was 0.1. Note that the ethanol was discharged in such a manner that the ethanol, when converted as gas, was about 3.6 times the ozone gas in volume ratio.

### [Example 4]

The hydroxyl radical fluorescence peak height was measured in the same manner as in example 1 except that an ethanol aqueous solution as a chemical agent was prepared by mixing the ethanol and water at a ratio of 3:7 in such a manner that the mole fraction of the ethanol was 0.3. Note that the ethanol was discharged in such a manner that the ethanol, when converted as gas, was about 11 times the ozone gas in volume ratio.

### [Comparative example 1]

The hydroxyl radical fluorescence peak height was measured in the same manner as in example 1 except that water having an alcohol mole fraction of 0 was used as the chemical agent.

### [Comparative example 2]

The hydroxyl radical fluorescence peak height was measured in the same manner as in example 1 except that an isopropanol aqueous solution as a chemical agent was prepared by mixing the isopropanol and water at a ratio of 1:1 in such a manner that the mole fraction of the isopropanol was 0.5. Note that the isopropanol was discharged in such a manner that the isopropanol, when converted as gas, was about 13 times the ozone gas in volume ratio.

### [Comparative example 3]

The hydroxyl radical fluorescence peak height was measured in the same manner as in example 1 except that an ethanol aqueous solution as a chemical agent was prepared by mixing the ethanol and water at a ratio of 1:1 in such a manner that the mole fraction of the ethanol was 0.5. Note that the ethanol was discharged in such a manner that the ethanol, when converted as gas, was about 19 times the ozone gas in volume ratio.

**[Table 1]**

| | Chemical agent | Hydroxyl radical fluorescence peak height (A.U.) | Alcohol volume ratio |
|---|---|---|---|
| Example 1 | Isopropanol aqueous solution (mole fraction 0.1) | 101 | 2.8 |
| Example 2 | Isopropanol aqueous solution (mole fraction 0.3) | 88 | 8.1 |
| Example 3 | Ethanol aqueous solution (mole fraction 0.1) | 127 | 3.6 |
| Example 4 | Ethanol aqueous solution (mole fraction 0.3) | 125 | 11 |
| Comparative example 1 | Water (mole fraction 0) | 67 | 0 |
| Comparative example 2 | Isopropanol aqueous solution (mole fraction 0.5) | 46 | 13 |
| Comparative example 3 | Ethanol aqueous solution (mole fraction 0.5) | 75 | 19 |

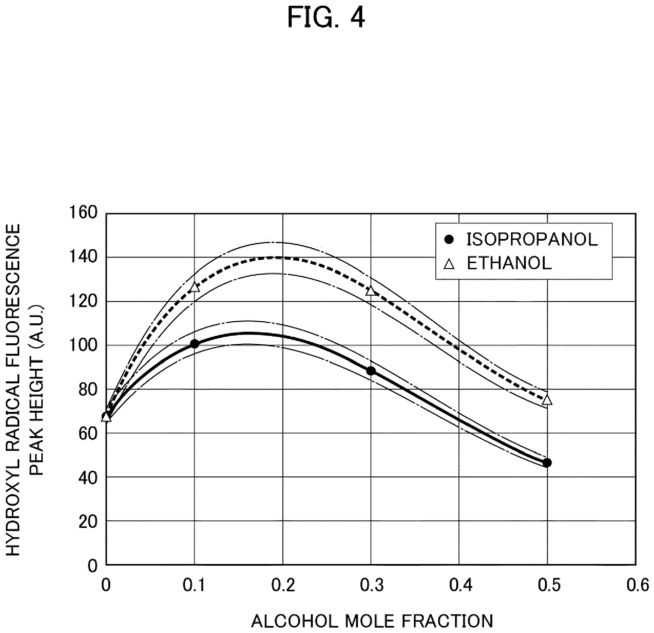

As illustrated in Table 1 and FIG. 4, in examples 1 to 4, an aqueous alcohol solution having a mole fraction between 0.05 and 0.35 is reacted with ozone gas. Note that lines of +5% and -5%, which are measurement errors of the hydroxyl radical fluorescence peak height, are added in Fig. 4. In these examples, the amount of generated hydroxyl radical increased compared with the case of causing ozone gas to react with water or an aqueous alcohol solution having a mole fraction of 0.5 as in comparative examples 1 to 3. For example, in the case of an ethanol aqueous solution (mole fraction of 0.1), the peak ratio of a hydroxyl radical is 1.9 times higher than that in the case of water, and the activity (sterilization performance) is about 2 times higher. The mechanism is not certain and is inferred from the reactivity of ozone gas. First, the presence of moisture facilitates the production of a hydroxyl radical through a reaction with ozone gas.

Since ozone gas is more reactive with an alcohol than water, the production of a hydroxyl radical is expected to be about 2 times higher when an aqueous alcohol solution as in examples 1 to 4 is made to react than when water is made to react. In contrast, if the mole fraction of an alcohol is too high, the hydroxyl radical generated will react with the alcohol and part of the hydroxyl radical generated will disappear. Thus, it is assumed that when the mole fraction is 0.5, the amount of a hydroxyl radical generated is low. Note that in this example, although the hydroxyl radical production amount when the concentration of ozone gas is 500 ppm is evaluated due to the convenience of the device, the same result is expected in a system having a low concentration of ozone gas, such as 0.1 ppm.

The volume ratio of the vaporized alcohol with respect to the ozone gas (alcohol volume ratio) is 2.8 in example 1 and 3.6 in example 3 where the hydroxyl radical concentration increases and the alcohol mole fraction is 0.1. In addition, the alcohol volume ratio is 8.1 in example 2 and 11 in example 4 where the alcohol mole fraction is 0.3. Thus, the amount of an alcohol added, when converted as gas, is preferably in the range up to 12 times the ozone gas in volume ratio.

The entire contents of Japanese Patent Application No. 2020-189387 (filed on November 13, 2020) are incorporated herein.

Although some embodiments have been described, it is possible to modify or alter the embodiments based on the contents of the above disclosure. All the components of the above-described embodiments and all the features described in the claims may be individually extracted and combined as long as they do not contradict each other.

The present disclosure can contribute, for example, to goal 3 "Ensure healthy lives and promote well-being for all at all ages" of the Sustainable Development Goals (SDGs) led by the United Nations.

### REFERENCE SIGNS LIST

- 1: Air cleaning system
- 10: First housing
- 15: Second housing
- 40: Ozone generation unit
- 50: Discharge unit
- 51: Third housing

## Claims

1. An air cleaning system comprising:
an ozone generation unit that generates ozone gas; and
a discharge unit that atomizes or vaporizes an aqueous alcohol solution and discharges the aqueous alcohol solution, wherein
a mole fraction of an alcohol included in the aqueous alcohol solution with respect to the aqueous alcohol solution is 0.05 or more and 0.35 or less, and
a hydroxyl radical is generated through a reaction of the ozone gas with the aqueous alcohol solution that has been atomized or vaporized.

2. The air cleaning system according to claim 1, wherein the alcohol includes at least one of ethanol or isopropanol.

3. The air cleaning system according to claim 1 or 2, wherein the alcohol is discharged in such a manner that the alcohol, when converted as gas, is 12 times or less the ozone gas in volume ratio.

4. The air cleaning system according to any one of claims 1 to 3, further comprising a first housing that houses the ozone generation unit and the discharge unit, wherein
the ozone gas and the aqueous alcohol solution that has been atomized or vaporized react inside the first housing.

5. The air cleaning system according to any one of claims 1 to 3, further comprising a first housing that houses the ozone generation unit and the discharge unit, wherein
the ozone gas and the aqueous alcohol solution that has been atomized or vaporized react outside the first housing.

6. The air cleaning system according to any one of claims 1 to 3, further comprising:
a second housing that houses the ozone generation unit; and
a third housing that houses the discharge unit, wherein
the ozone gas and the aqueous alcohol solution that has been atomized or vaporized react outside the second housing and the third housing.

7. The air cleaning system according to any one of claims 1 to 6, wherein an ozone gas concentration in an external space is controlled to be less than or equal to 0.1 ppm.

8. A method for cleaning air comprising:
a step of generating ozone gas;
a step of atomizing or vaporizing an aqueous alcohol solution and discharging the aqueous alcohol solution; and
a step of generating a hydroxyl radical through a reaction of the ozone gas with the aqueous alcohol solution that has been atomized or vaporized, wherein
a mole fraction of an alcohol included in the aqueous alcohol solution with respect to the aqueous alcohol solution is 0.05 or more and 0.35 or less.
